# EUROPEAN PATENT APPLICATION

(11) **EP 3 763 341 A1**
(43) Date of publication of application: **13.01.2021**
(21) Application number: 19185897.6
(22) Date of filing: 11.07.2019
(51) Int. Cl.: A61F 7/12, A61B 1/303, A61H 19/00, A61H 9/00, A61B 17/34

(54) **A DEVICE FOR TREATING VAGINAL ATROPHY**

(71) Applicant: National University of Ireland, Galway, Galway (IE)
(72) Inventor: Newall, Paula, Galway, Co. Galway H91 T6TC (IE)
(74) Representative: Johnson, Carrie-Anne Louise

(57) **Abstract**

A device (1, 20) to treat vaginal atrophy comprises a treatment module (2, 21) configured for insertion into the vagina and having a microtrauma module configured to deliver mechanical microtrauma therapy to a wall of the vagina when inserted. The microtrauma module is configured to deliver a mechanical microtrauma therapy to the wall of the vagina selected from negative pressure, needle puncture, clamping, heating, cooling, friction or rolling to the wall of the vagina. In one preferred embodiment, the microtrauma module is configured to deliver negative pressure therapy to the wall of the vagina.

## Description

### Field of the Invention

The present invention relates to a device for treating vaginal atrophy. Also contemplated are methods of treating vaginal atrophy/dryness.

### Background to the Invention

Vulva and/or Vaginal Atrophy (VVA/VA) is a chronic & progressive condition because of a reduction or absence of oestrogen. Vaginal Atrophy (VA) is a chronic and progressive condition that affects approximately 80% of women because of hormonal changes at some point during their lifetime. It results from reduced oestrogen levels that cause the vagina and surrounding tissue to become dry, thin and inflamed. Symptoms include chronic dryness, itching, irritation and pain. Studies have shown it impairs a woman's quality of life, affects intimate relationships and makes it uncomfortable to sit, stand, exercise, urinate or even work. VA is a prevalent condition and is most common in women in the menopausal age range and Breast Cancer Survivors (BCSs). It is estimated that by 2020, there will be 50 million menopausal women in the US and by 2025 there will be 1.1 billion women in the menopausal range across the world suffering from VA1,11. Additionally, there are 3.1 million BCSs suffering with VA. There is currently note an o affordable, safe, and effective treatment for VA. AVeta Medical seeks to address this clinical need.

When a woman experiences the menopause, oestrogen production is decreased by 70%. With VA, the tissues of a woman's vagina no longer work in their normal, healthy way. The lining of the vagina begin to shrink and walls become thinner and drier that makes for an uncomfortable dry sensation that affects a woman's quality of life. The vaginal epithelium becomes thinner, elasticity is reduced, vaginal secretions decrease and blood flow is reduced.

More than 60% of BCSs become postmenopausal after chemotherapy and radiation treatment and consequently BCSs experience VA6-8. The chemotherapy and radiation causes a reduction in oestrogen hence induce early menopause. Additionally, the cancer blocker and endocrine drugs that women take to remain cancer free exacerbate the symptoms of VA owing to the oestrogen suppression effect of these therapies that worsens the symptoms of itchiness, pain, dryness around the vagina area. Additionally, VA is often the most poorly addressed side effect for BCS on adjuvant endocrine therapy.

It is an object of the invention to overcome at least one of the above-referenced problems.

### Summary of the Invention

The present invention addresses the need for a treatment of vaginal atrophy that does not involve side effects, can be self-administered, is suitable for use with all women, not just menopausal women or breast cancer patients, and that addresses the underlying cause of the indication without causing irreversible or serious damage to the vaginal epithelium. The solution is based on a device configured to be inserted into the vagina and actuated to cause mechanical microtrauma to the wall of the vagina. Mechanical microtrauma refers to small deformations to the vaginal wall due to physical interaction between the device and wall that are sufficient to cause inflammation-induced angiogenesis and stimulate proliferation of new endothelial cells and/or generation of collagen matrix. The micro-deformations may include micro tears, punctures, holes, stretches, and cracks in the vaginal wall. Various method of applying mechanical microtrauma to the wall of the vagina using an insertable device are described herein.

In a first aspect, the invention provides a device to treat vaginal atrophy comprising a treatment module configured for insertion into the vagina and having a microtrauma module configured to deliver mechanical microtrauma therapy to a wall of the vagina when inserted.

In one embodiment, the microtrauma module is configured to deliver a mechanical microtrauma therapy to the wall of the vagina selected from negative pressure, needle puncture, clamping, heating, cooling, friction or rolling to the wall of the vagina.

In one embodiment, the microtrauma module is configured to deliver negative pressure therapy to the wall of the vagina. Negative pressure therapyhas been suggested previously as a treatment of wounds (Cipolla et al. (Negative pressure wound therapy: Unusual and innovative applications. OPUS 12 Scientist 2008;2(3):15-29) and Saxena et al (2004 Vacuum-assisted closure: Microdeformations of wounds and cell proliferation. Plastic and Reconstructive Surgery, 114(5), 1086-1096)). The Applicants have discovered that negative pressure therapy is a particularly suitable method of causing mechanical microtrauma to the wall of the vagina. The application of negative pressure to the wall of the vagina stimulates proliferation of new endothelial cells and collagen matrix that will thicken the vaginal wall, reduce atrophy, promote angiogenesis and improve the moisture retaining ability of cells in the vaginal wall, reducing dryness. It is particularly applicable as a therapy for breast cancer patients, and menopausal women, for whom vaginal atrophy is a recognised side-effect. A key molecule involved in skin and tissue moisture is hyaluronic acid (HA) that has a unique capacity in retaining water. The synthesis of HA increase in response to inflammation or trauma to the vagina tissue (i.e. the negative pressure will induce a controlled beneficial micro-trauma). HA regulates several aspects of tissue repair including activation of inflammatory endothelial cells to enhance angiogenesis to form new blood vessels from existing ones. The synthesis of HA is decreased with aging which in turn reduces oestrogen production.

In one embodiment, the microtrauma module comprises a plurality of small apertures in fluidic connection with a vacuum pump. 12. The number and size of the apertures employed, and the arrangement of apertures on the microtrauma module, is variable. For example, the apertures may have a maximum dimension of 1-6 mm. The apertures may be arranged circumferentially around the microtrauma module, or arranged bilaterally in discrete arrays on the microtrauma module. Any number of apertures may be employed, for example at least 10, or 10-100.

Generally, when the device is configured to deliver mechanical microtrauma therapy by means of negative pressure therapy, the device will include a vacuum pump operatively connected to the microtrauma module to apply negative pressure to the wall of the vagina through the small apertures. The vacuum pump may be configured to generate a negative pressure of 50-800 mmHg during use, and may be configured for user adjustment.

In one embodiment, the treatment module comprises a proximal handle part and a distal treatment part. The distal treatment part is dimensioned for insertion into the vagina, and delivery of mechanical microtrauma therapy to the wall of the vagina. The proximal handle part is configured for being held by a user during use. The treatment module is generally an elongated body in which the distal treatment part of the treatment module is optionally cranked with respect to the proximal handle part (for example by about 2-45 degrees).

In one embodiment, the distal treatment part is detachable from the proximal handle part. This allows the distal treatment part to be configured for single use, and disposed of after use, while retaining the handle part of multiple uses.

In one embodiment, the device comprises a pressure sensor configured to determine an operational pressure of the negative pressure therapy being applied to the wall of the vagina.

In one embodiment, the device comprises an actuation/controller module. This module may comprise actuation means for the microtrauma module (for example the vacuum pump in the case of negative pressure therapy, or a motor for actuation of microneedles). This module may also include one or more of a graphical user interface, a processor, and a sensor. The actuation/controller module may be located within the microtrauma module (for example the handle part), or it may be located remotely, in a separate base station.

In one embodiment, the actuation/controller module comprises a vacuum pump, a negative pressure sensor, and a processor configured to:
receive data relating to the operational pressure from the negative pressure sensor;
compare the detected operational pressure with a reference pressure; and
modify the operational pressure to achieve a target pressure when a sub-optimal operational pressure is detected.

In one embodiment, the device comprises a graphical user interface operatively connected to the actuation/controller module, and configured to graphically display a parameter of the mechanical microtrauma therapy (for example the negative pressure being applied, the length of the treatment period, the time elapsed of the treatment period, the time remaining of the treatment period, etc).

In one embodiment, the device comprises a timer configured to switch off the device after a pre-defined treatment period.

In one embodiment, the treatment module includes an imaging device (for example a video or CCD camera) and optionally an illumination system, configured for imaging the wall of the vagina during use. The device may be configured to display images from the imaging device on the graphical user interface.

In one embodiment, the treatment module includes a moisture sensor configured to sense moisture of the vaginal wall. In one embodiment, the device comprises a processor configured to receive moisture data from the moisture sensor, and process or store the data. The processor may be configured to display data relating the moisture on a graphical user interface forming part of the device, or wirelessly relay the data to a remote computational device.

In one preferred embodiment, the actuation/controller module is a remote actuation/controller module (i.e. provided as a remote base station), operatively connectable to the treatment module and comprising actuating and/or controlling means for the microtrauma module. The actuation/controller module may be operatively connected to the treatment module by, for example, a flexible lead or tubing/conduit. In one embodiment, the treatment module is detachable from the remote actuation/controller module. This configuration allows for use of a disposable treatment module, which can be detached from the remote actuation/controller module after use, and disposed of.

In one embodiment, the device (for example the processor) may include communication means for relaying data wirelessly to a remote computational device (for example a mobile phone) via a communication network. The communication network can be the internet, an intranet, or any wired or wireless communication network. For example, the communication network may include a Mobile Communications (GSM) network, a code division multiple access (CDMA) network, 3rd Generation Partnership Project (GPP), an Internet Protocol (IP) network, a wireless application protocol (WAP) network, a WiFi network, or an IEEE 802.11 standards network, as well as various communications thereof. Other conventional and/or later developed wired and wireless networks may also be used.

In one embodiment, the device, particularly the treatment module, and more particularly the distal treatment head, is formed from a soft lubricious material.

In another aspect, the invention provides a method of treatment of vaginal atrophy in a mammal, especially a human, comprising the steps of administering mechanical microtrauma to a wall of the vagina of the mammal, optionally using an insertable device comprising a microtrauma module configured to deliver mechanical microtrauma therapy to a wall of the vagina when inserted. In one embodiment, the insertable device is the device of the invention.

In one embodiment, the method comprises administering mechanical microtrauma therapy to the wall of the vagina for a treatment period. The duration of a treatment period may be 1-90, 1-60, 1-30, 5-90, 5-60, or 5-30 minutes. In one embodiment, the mammal is treated once every 1, 2, 3, 4, 5, 6, or 7 days, for weekly, fortnightly or monthly. In one embodiment, the mammal is suffering from vaginal atrophy. In one embodiment, the mammal is suffering from vaginal dryness.

In one embodiment, the method comprises the steps of positioning the treatment module at a first position in the vagina, and applying a first dose of mechanical microtrauma therapy, and then re-positioning the treatment module at a second position in the vagina, and applying a second dose of mechanical microtrauma therapy. In one embodiment, re-positioning comprises rotating the device about a longitudinal axis. In another embodiment, re-positioning comprising adjusting the depth of the device.

Other aspects and preferred embodiments of the invention are defined and described in the other claims set out below.

### Brief Description of the Figures

**Figure 1** illustrates the anatomy of the vagina, womb and uterus.
**Figure 2** illustrates how the device of the invention is employed: (A) the treatment module forming part of the device is inserted into the vagina and mechanical microabrasion therapy is delivered by deployment of a plurality of needle into the wall of the vagina; and (B) the treatment module is inserted into the vagina and mechanical microabrasion therapy is delivered by application of negative pressure therapy to the wall of the vagina.
**Figure 3** illustrates a vacuum therapy treatment module forming part of a device of the invention in elevational view (A) and plan view (B).
**Figure 4** illustrates a vacuum therapy treatment module for part of a device of the invention in elevational view: (A) the small apertures are provided as an array disposed on opposite sides of the wall of the module; and (B) the small apertures are provided as a single array circumferentially arranged around of the wall of the module;
**Figure 5** illustrates the device of the invention comprising the treatment module, a remote actuation/controller module, and a lead providing operational communication between the treatment module and remote module.
**Figure 6** is a block diagram illustrating the design of one embodiment of the device of the invention.
**Figures 7A and 7B** are illustrations of a device according to an alternative embodiment of the invention in which the actuation/controller module and power source is located in the handle.
**Figure 8** is an illustration of a system comprising a device of the invention and a mobile phone.
**Figure 9** illustrates a device of the invention and a storage case, resting on a bed-side locker.
**Figure 10** illustrates one method of how the flexible lead may connect to the actuator/controller.
**Figure 11** illustrates a device of the invention.
**Figure 12A to 12C** illustrate information that can be graphically illustrated on the LCD screen.
**Figure 13** illustrates an embodiment of the actuator/controller, similar to the embodiment of Figure 10.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and vice versa. The term "a" or "an" used in relation to an entity is to be read to refer to one or more of that entity. As such, the terms "a" (or "an"), "one or more," and "at least one" are used interchangeably herein.
As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.
As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.
As used herein, the term "vaginal atrophy" refers to the chronic & progressive condition caused by a reduction or absence of oestrogen. It causes the vagina and tissue near it to become dry, thin & inflamed. Vaginal atrophy is prevalent in menopausal women, but more so in postmenopausal Breast Cancer Survivors (BCS). VA describes a range of symptoms including vaginal dryness, irritation, pain & urinary incontinence & affects a large percentage of menopausal women. This condition disproportionately affects BCS who experience premature menopause as a result of their cancer treatments. BCS who receive systemic endocrine therapy (e.g., aromatase inhibitors and Tamoxifen) also experience more severe VA symptoms owing to the oestrogen suppression effect of these therapies. The device and methods of the invention are to treat vaginal atrophy, and vaginal dryness.
As used herein, the term "treatment" or "treating" refers to an intervention (e.g. the administration of an agent to a subject) which cures, ameliorates or lessens the symptoms of a disease or removes (or lessens the impact of) its cause(s). In this case, the term is used synonymously with the term "therapy". The treatment may be causal or symptomatic. Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".
As used herein, an effective amount or a therapeutically effective amount of a negative pressure treatment defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount (pressure and/or length of treatment) will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.
In the context of treatment and effective amounts as defined above, the term subject (which is to be read to include "individual", "animal", "patient" or "mammal" where context permits) defines any subject, particularly a mammalian female subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.
"Treatment module" refers to a part of the device of the invention that is configured for at least partial insertion into the vagina and includes a microtrauma module to deliver mechanical microtrauma therapy to a wall of the vagina when inserted. The module (or at least the part that is inserted into the vagina) will generally have a smooth, autraumatic shape, and a surface comprising the microtrauma module configured to abut the wall of the vagina when inserted. The treatment module may have a distal (transvaginal) part configured for insertion into the vagina ("distal treatment part"), and a proximal part configured for being held by a user during use ("proximal handle part").
"Microtrauma module" means part of the treatment module that is configured to deliver mechanical microtrauma therapy to a wall of the vagina, when the treatment module is inserted into the vagina.
"Mechanical microtrauma" refers to small deformations to the vaginal wall due to physical interaction between the microtrauma module and wall that are sufficient to cause inflammation-induced angiogenesis and stimulate proliferation of new endothelial cells and/or generation of collagen matrix. The micro-deformations may include micro tears, punctures, holes, stretches, and cracks in the vaginal wall. On embodiment of mechanical microtrauma employs negative pressure therapy which is describe in more detail below. However, other methods of delivering mechanical microtrauma therapy to the wall of the vagina are envisaged. For example, the microtrauma module may comprise needles (or more preferably microneedles) configured for deployment during use, to create microabrasions in the vaginal wall. The microneedles may be actuated for deployment from a retracted delivery position within the device to a deployed position during use where the microneedles penetrate the wall of the vagina. In another embodiment, the microtrauma module may comprise means for stretching the wall of the vagina to create micro tears or fissures in the wall, for example means for gripping a section of the wall at each end of the section, and then stretching the wall. In another embodiment, the use of heating or cooling may be employed to generate mechanical microtrauma in the wall of the vagina. In another embodiment, the mechanical microtrauma module may comprise means for applying friction or mechanical deformation (rolling) to the wall of the vagina.
"Negative pressure therapy" refers to the process of forming micro-deformations in the wall of the vagina by the application of a vacuum to the wall of the vagina, where the micro-deformations are sufficient to stimulate proliferation of new endothelial cells and collagen matrix that will thicken the vaginal wall, reduce atrophy and improve the moisture retaining ability of cells in the vaginal wall, reducing dryness. One embodiment of the device of the invention applies negative pressure therapy to an area of the wall of the vagina by means of a plurality of small apertures, typically an array of small closely-packed apertures, that are fluidically connected to a vacuum pump in the device. The application of negative pressure therapy to wound healing is described in the literature, for example Cipolla et al. (Negative pressure wound therapy: Unusual and innovative applications. OPUS 12 Scientist 2008;2(3):15-29) and Saxena et al (2004 Vacuum-assisted closure: Microdeformations of wounds and cell proliferation. Plastic and Reconstructive Surgery, 114(5), 1086-1096). Negative pressure wound therapy facilitates healing by improving the rate of angiogenesis, endothelial proliferation, capillary blood flow and decreasing interstitial edema (amongst other things). This approach is applicable to the vaginal wall where it causes a similar beneficial micro-trauma effect. The negative pressure produces a controlled, beneficial micro-trauma to the vaginal wall to induce a beneficial inflammation cascade of angiogenesis and neo-collagenesis (collagen renewal) which is recognised molecular pathway. Negative pressure therapy is supported by literature to induce angiogenesis & endothelial proliferation that are the mechanisms of action required to rejuvenate the vaginal wall to address vaginal atrophy. Vaginal lubrication originates from the lamina propria, which contains elastic fibres, blood vessels, lymphatic & nerves, as well as glands that secrete mucus and serous fluids. The lamina propria of the atrophic vagina has decreased extracellular matrix components, reduced vascularization & water-retaining capacity. The device and methods of the invention induceangiogenesis of the lamina propia to create micro-vascularization & new vessel formation. This in turn will produce moisture & lubrication, simultaneously collagen remodelling of the connective tissue & elastin fibres of the vaginal wall & restoring the vaginal mucosa & rehydrating the vaginal walls. The synthesis of HA increase in response to inflammation or trauma to the vagina tissue (i.e. the tip with the perforations will induce a controlled beneficial micro-trauma). HA regulates several aspects of tissue repair including activation of inflammatory endothelial cells to enhance angiogenesis to form new blood vessels from existing ones. The synthesis of HA is decreased with aging which in turn reduces oestrogen production.
"Small apertures" should be understood to mean holes that are sufficiently small to deliver effective negative pressure to the wall of the vagina. Typically, the holes have a maximum diameter of about 1-3 mm, 2-3 mm, or preferably about 2.5 mm. Typically the holes are circular or oval, although other shapes of holes may be employed for example square, rectangular, provided that they are sufficiently small.
"Remote actuation/controller module" refers to an actuation/controller module that is separate from the treatment module and generally connected by a connecting lead. It is also referred to herein as a remote "base station". The remote actuation/controller module generally includes means for actuating the mechanical microtrauma module, for example a pump, motor, heater, a graphical user interface, and a processor for receiving data relating the operation of the treatment module or the treatment.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

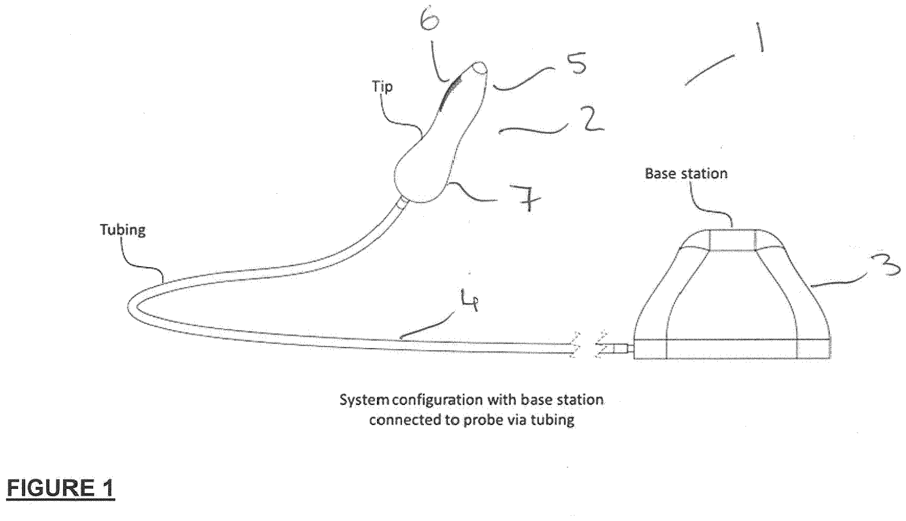

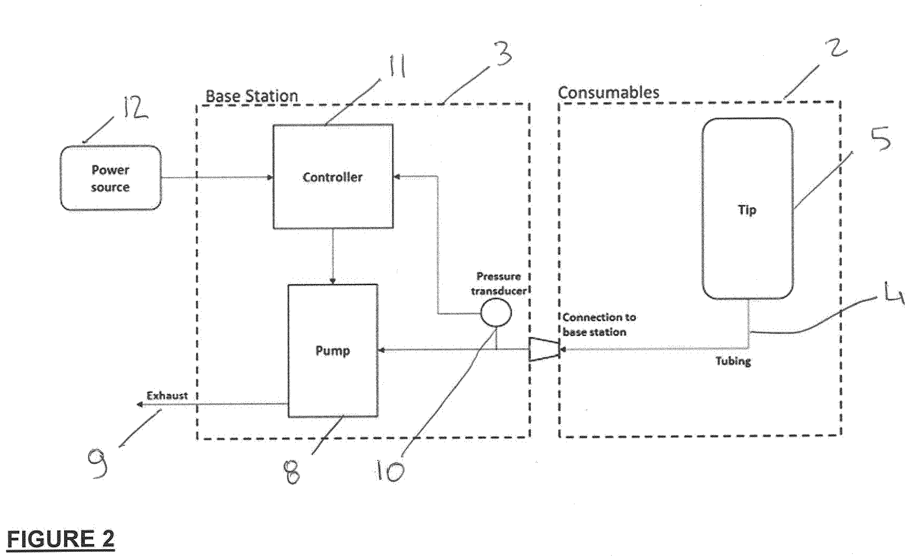

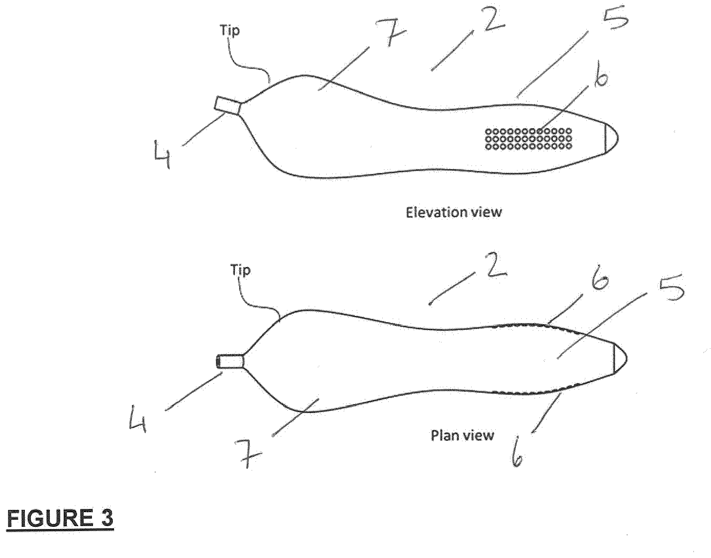

Referring to the drawings, and initially to Figures 1 to 3, there is illustrated one embodiment of a device on the invention, indicated generally by the reference numeral 1. In this embodiment, the device is configured for delivery of mechanical microabrasion therapy by means of negative pressure therapy. The device 1 comprises a therapeutic module 2, a remote actuator/controller module 3, and a flexible lead 4 providing operational communication between the therapeutic module 2 and remote module 3. The therapeutic module 3 comprises a distal treatment part 5 having a curved atraumatic shape, and a microtrauma module provided in the form of two arrays of small apertures 6 disposed on opposite sides of a sidewall of the distal part, and a proximal part 7 configured to be held by the user during use of the device. The actuation/controller module 3 comprises a vacuum pump 8 fluidically connected via the lead 4 to the array of small apertures 6, and to an exhaust 9, a pressure transducer 10 configured to detect in real time the vacuum being drawn by the pump 8, and a processor 11 configured to receive pressure data from the pressure transducer 10. A power source 12 is operatively connected to the processor, pump and pressure transducer, which power source may be mains electricity or a batter (not shown). The processor is configured to receive pressure data from the transducer 8, and process the data. The processor may be pre-programmed to apply a level of negative pressure therapy, and may be configured to adjust the pump to module the pressure according to pressure readings received from the pressure transducer. The processor may be operatively connected to a graphical user interface (GUI) to display pressure readings on the GUI.

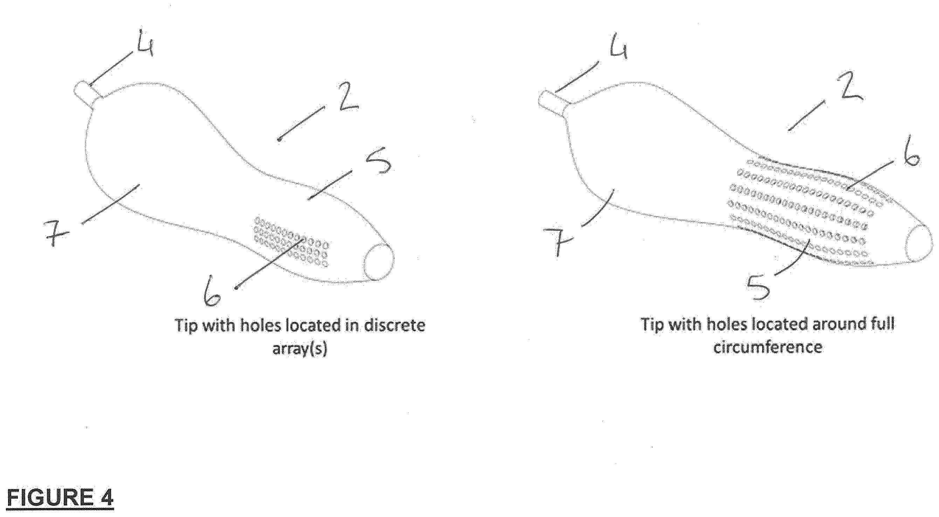

Figure 4 illustrates two embodiments of the treatment module in which the small apertures are provided as an array disposed on opposite sides of the wall of the module (A), and the small apertures are provided as a single array circumferentially arranged around of the wall of the module (B). It will be appreciated that the small apertures may be provided in other arrangements, including multiple arrays of apertures.

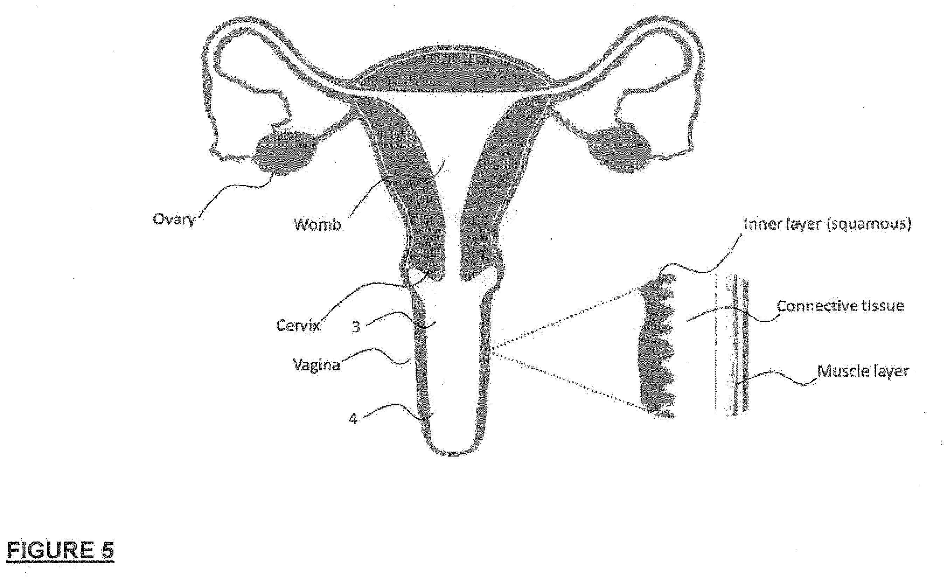

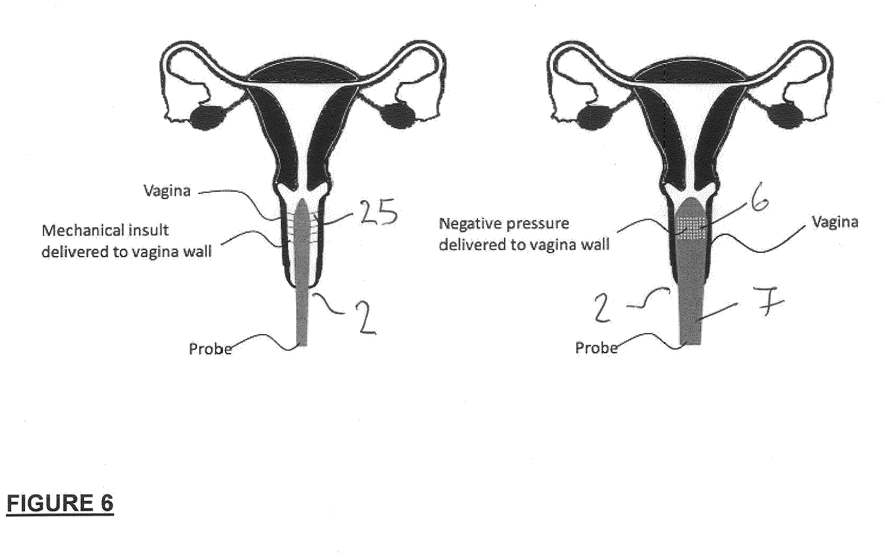

The use of the device of the invention is illustrated in Figures 5 and 6, where Figure 5 illustrates the anatomy of the womb and vagina of a female human, and Figure 6 illustrates the distal treatment part of the treatment module of the device inserted into the vagina and actuated to administer mechanical microtrauma therapy. In Figure 6B (image on right), the device comprises a circumferential array of small apertures configured to deliver negative pressure therapy to the wall of the vagina in contact with the apertures. In Figure 6A (image on left), the device comprises an array of deployable needles 25 configured for deployment into the wall of the vagina. In both cases, the treatment results in the creation of micro-deformations in the wall of the vagina that are sufficient to cause angiogenesis and stimulate proliferation of new endothelial cells and/or generation of collagen matrix, and thereby treating vaginal atrophy.

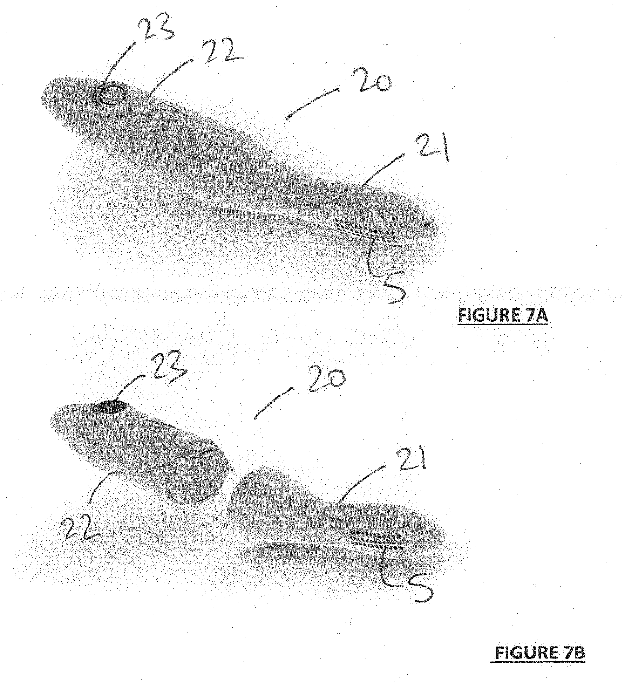

Referring to Figures 7A and 7B, an alternative embodiment of the device of the invention is illustrated, in which parts identified with reference to the previous embodiments are assigned the same reference numerals. In this embodiment, indicated by the reference numeral 20, the device is provided as a single elongated body having a distal treatment part 21 with arrays of apertures 5 and a proximal handle part 22, which parts are configured for detachment. The handle part 22 includes a vacuum pump, processor, pressure sensor, a battery (not shown), and an on/off button 23. The distal treatment part is a single use, disposable, part, which can be discarded after use. This embodiment of the device may be provided in a modular format, comprising a single handle part, and a number of disposable distal treatment parts.

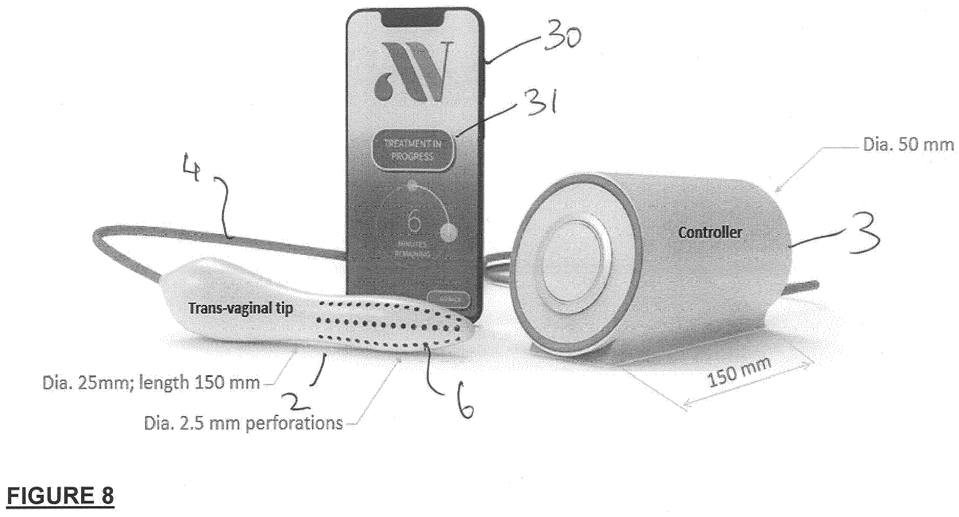

Referring to Figure 8, there is illustrated a device of the invention in which parts identified with reference to the previous embodiments are assigned the same reference numerals. The device comprises a treatment module 2 and a remote actuator controller 3 connected by a lead 4, as described previously. The actuator/controller is configured to relay data to a mobile phone 30, and the phone comprises downloadable software (i.e. an "app") configured to receive and process data received from the actuator/controller, and display information relating to the operation of the device and the therapy on the phone screen 31. In the illustration, the phone provides information relation to the status of the therapy (i.e. treatment in progress) and in relation in relation to the treatment time remaining.

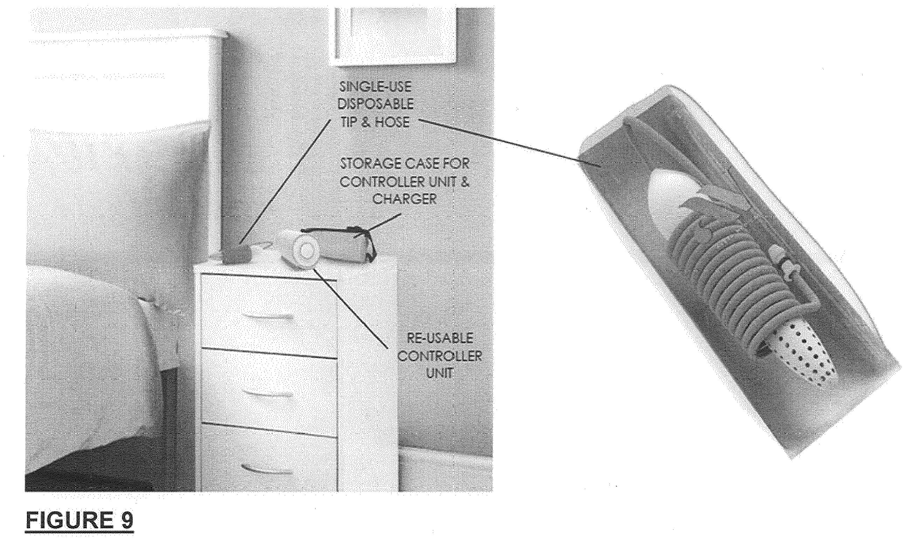

Referring to Figure 9, there is illustrated a device of the invention and a storage case, resting on a bed-side locker. The device comprises a remote re-usable actuator/controller, a disposable treatment module and flexible lead. A storage case of the actuator controller is provided, along with a storage case for the treatment module and flexible lead.

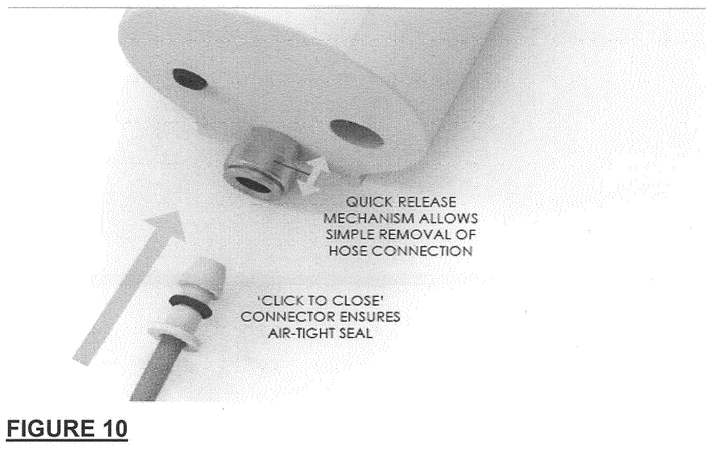

Referring to Figure 10, there is illustrated one method of how the flexible lead may connect to the actuator/controller, having a friction-fit "click-to-close" connecting mechanism, and a quick release mechanism to allow for easy and fast disconnection of the lead and the actuation controller.

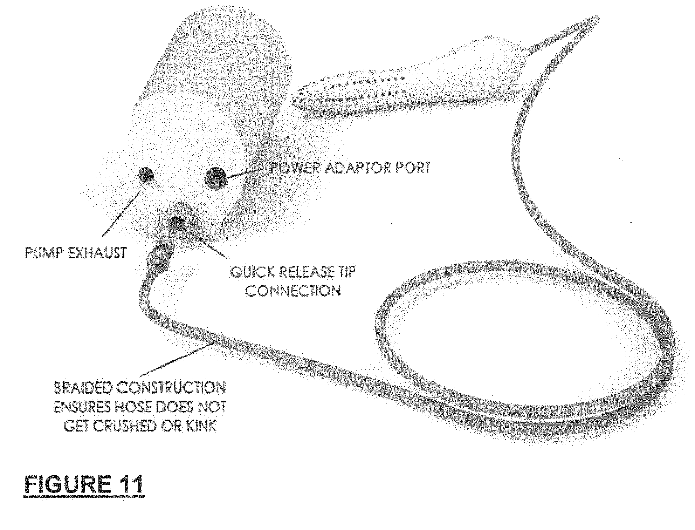

Referring to Figure 11, there is illustrated a device of the invention, showing an end face of the actuator/controller having a port for receipt of the flexible lead, a pump exhaust vent, and a power adaptor port. Also illustrated in a flexible lead comprising a braided sheath which helps avoid crushing or kinking of the lead.

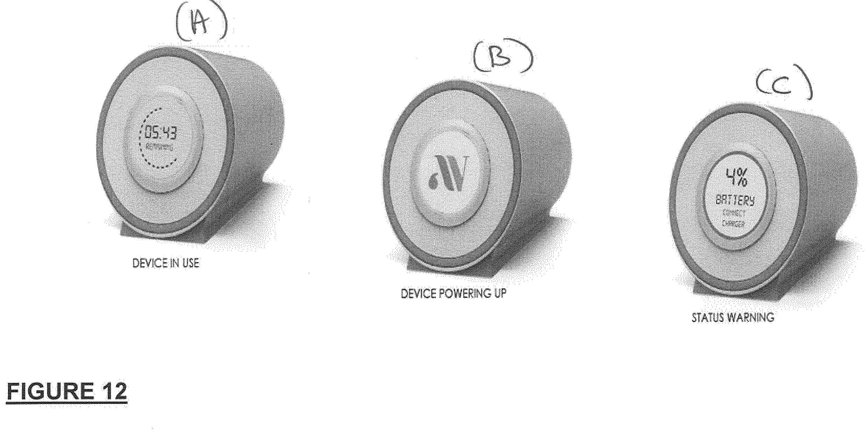

Referring to Figures 12A to 12C, there is illustrated information that can be graphically illustrated on the LCD screen including information relating to a treatment period (A), information relating to the operational status of the device (B), and information relating to the remaining battery power of the device (C).

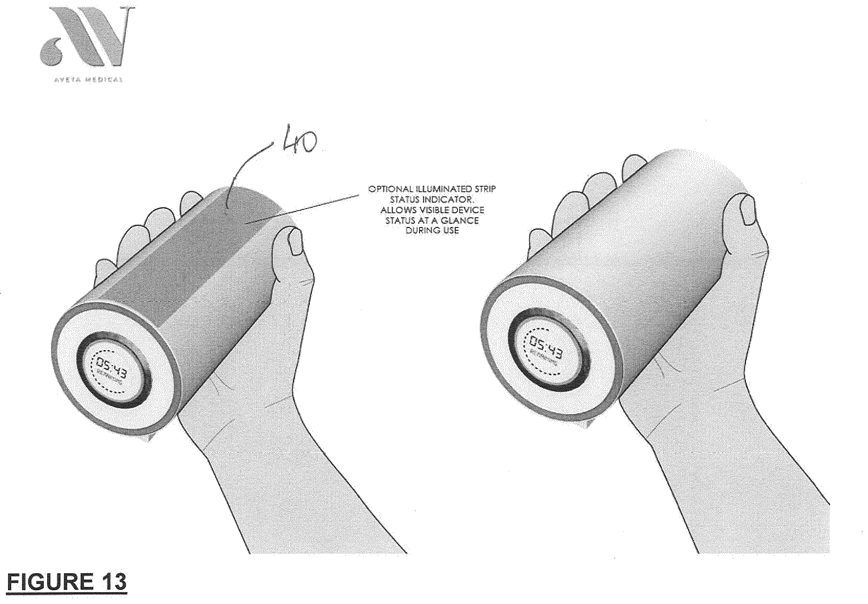

Referring to Figure 13, there is illustrated an embodiment of the actuator/controller, similar to the embodiment of Figure 10, in which the actuator/controller comprises a graphical display disposed on a side of the actuator (in this case a strip of illumination 40) that can indicate the device status at a glance.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A device (1, 20) to treat vaginal atrophy comprising a treatment module (2, 21) configured for insertion into the vagina and having a microtrauma module configured to deliver mechanical microtrauma therapy to a wall of the vagina when inserted.

2. A device as claimed in Claim 1, in which the microtrauma module is configured to deliver a mechanical microtrauma therapy to the wall of the vagina selected from negative pressure, needle puncture, clamping, heating, cooling, friction or rolling to the wall of the vagina.

3. A device according to Claim 2, in which the microtrauma module is configured to deliver negative pressure therapy to the wall of the vagina.

4. A device according to Claim 3, in which the microtrauma module comprises a plurality of small apertures (6) in fluidic connection with a vacuum pump.

5. A device according to Claim 1, in which the treatment module comprises a proximal handle part (7) and a distal treatment part (5).

6. A device according to Claim 5, in which the distal treatment part (21) is detachable from the proximal handle part (22), and in which the distal treatment part is disposable.

7. A device as claimed in any preceding Claim, in which the device comprises a remote actuation/controller module (3) operatively connectable to the treatment module (2) and comprising actuating and/or controlling means for the microtrauma module.

8. A device according to Claim 7, in which the treatment module (2) is detachable from the remote actuation/controller module (3), and is disposable.

9. A device according to Claim 7 or 8, in which the actuation/controller module (3) comprises a graphical user interface.

10. A device according to any of Claims 3 to 9, in which the device is configured to generate a negative pressure of 50-800 mmHg during use.

11. A device according to any preceding Claim, in which the treatment module comprises a moisture sensor.

12. A device according to any of Claims 4 to 11, in which the plurality of small apertures (6) are arranged circumferentially around the microtrauma module.

13. A device according to any of Claims 4 to 11, in which the plurality of small apertures (6) are arranged bilaterally in discrete arrays on the microtrauma module.

14. A device according to any of Claims 3 to 15, in which the device comprises a pressure sensor (10) configured to determine an operational pressure of the negative pressure therapy being applied to the wall of the vagina, and a processor (11) configured to receive data relating to the operational pressure from the pressure sensor, compare the detected operational pressure with a reference pressure, and modify the operational pressure to achieve a target pressure when a sub-optimal operational pressure is detected.

15. A device according to any preceding Claim, in which the device comprises a timer configured to switch off the device after a pre-defined treatment period, and optionally a camera and optionally an illumination system configured for imaging the wall of the vagina during use.
